## Europäisches Patentamt

## European Patent Office

## Office européen des brevets

(11) Veröffentlichungsnummer: **0 094 341**
**B1**

(12) # EUROPÄISCHE PATENTSCHRIFT

(45) Veröffentlichungstag der Patentschrift:
**13.01.88**

(51) Int. Cl.⁴: **A 61 N 1/36, H 02 M 3/07**

(21) Anmeldenummer: **83730047.4**

(22) Anmeldetag: **11.05.83**

(54) Herzschrittmacher.

(30) Priorität: **11.05.82 DE 3218117**

(43) Veröffentlichungstag der Anmeldung:
**16.11.83 Patentblatt 83/46**

(45) Bekanntmachung des Hinweises auf die Patenterteilung:
**13.01.88 Patentblatt 88/2**

(84) Benannte Vertragsstaaten:
**DE FR GB IT NL SE**

(56) Entgegenhaltungen:
**EP-A-0 014 833**
**EP-A-0 029 479**
**DE-A-2 701 457**
**DE-A-2 939 233**
**DE-A-2 939 255**
**DE-A-3 011 086**
**US-A-3 726 285**
**US-A-4 015 609**

**IBM CORPORATION, "IBM Technical Disclosure Bulletin", Vol. 25, Nr. 2, Juli 1982, Seiten 565, 566**

(73) Patentinhaber: **BIOTRONIK Mess- und Therapiegeräte GmbH & Co Ingenieurbüro Berlin, Sieversufer 8, D-1000 Berlin 47 (DE)**

(72) Erfinder: **Freiherr v. Nettelhorst, Herwig, Dr.- Ing., Pössneckerstrasse 30, D-1000 Berlin 45 (DE)**
Erfinder: **Rossdeutscher, Wolfram, Dipl.- Ing., Wilhelm- Pasewaldt- Strasse 23, D-1000 Berlin 42 (DE)**

(74) Vertreter: **Christiansen, Henning, Dipl.- Ing., CHRISTIANSEN & NINNEMANN Dietrich- Schäfer- Weg 21, D-1000 Berlin 41 (DE)**

**Beschreibung**

Die Erfindung betrifft einen Herzschrittmacher oder ein Elektrostimulationsgerät der im Oberbegriff des Anspruch 1 angegebenen Art. Ein derartiges Elektrostimulationsgerät ist aus der EP-A1-0 029 479 bekannt.

Bei einem bekannten aus der US-A-3 726 285 Schrittmacher kann die Spannungsvervielfacherschaltung zur Versorgung der die Stimulationsimpulse erzeugenden Ausgangsschaltung über einen magnetischen Reedschalter von einer Verdopplerschaltung in eine Verdreifacherschaltung umgeschaltet werden.

Diese Lösung hat den Nachteil, daß nur zwei unterschiedliche Ausgangsamplituden einstellbar sind.

Weiterhin ist aus der US-A-4 015 609 eine Schaltung bekannt, welche einen Spannungsvervielfacher enthält und eine herkömmliche Stabilisatorschaltung mit einem Transistor als Regelglied. Diese Schaltung weist den Nachteil auf, daß die Regelung wegen der an dem Regelglied abfallenden Leistung mit beträchtlichen Verlusten behaftet ist, so daß die Betriebszeit des Schrittmachers unnötig herabgesetzt ist.

Aus der DE-A1-29 39 255 ist eine entsprechende Schaltung bekannt, bei der ein Ladungsspeicher impulsweise entsprechend einem vorgegebenen funktionellen Verlauf in Abhängigkeit von einem Soll-/Istwert-Vergleich auf- oder entladbar ist. Auch bei dieser Lösung treten Energieverluste auf, die gerade bei batteriebetriebenen implantierbaren Systemen vermieden werden sollen.

Der Erfindung liegt die Aufgabe zugrunde, eine Schaltung für einen Herzschrittmacher bzw. ein entsprechendes Stimulationsgerät, der vorgenannten Gattung anzugeben, bei der die Spannung der Stimulationsimpulse in weiten Grenzen stufenlos veränderlich ist. Die durch die Schaltung zur Änderung der Ausgangsamplitude hervorgerufenen Verluste sollen dabei möglichst gering sein.

Diese Aufgabe wird durch einen Herzschrittmacher mit den im Anspruch 1 angegebenen Merkmalen gelöst.

Durch die Möglichkeit der Sperrung der Ladungsimpulse des Kondensators, der die Versorgungsspannung für die Stimulationsendstufe bereitstellt, ist eine Spannungsregelung praktisch ohne wesentliche Verluste möglich, wobei in vorteilhafter Weise der zwischen aufeinanderfolgenden Stimulationsimpulsen zur Verfügung stehende Zeitraum benutzt wird, um für den jeweils nächsten Stimulationsimpuls wieder die notwendige Versorgungsspannung zur Verfügung zustellen. Die Schaltung bildet eine getaktete Regelung mit deren vorteilhafter Energiebilanz, wobei der pulsweise Betrieb gleichzeitig eine Eigenschaft der Teilschaltung darstellt, welche zur Anhebung der Ausgangsspannung über die Versorgungsspannung hinaus vorgesehen ist. (Unter Vervielfacherschaltung soll hier somit eine Schaltung verstanden werden, welche in taktweisem Betrieb eine Heraufsetzung der Versorgungsspannung - auch um nicht ganzzahlige Faktoren ermöglicht.)

Durch den verwendeten Komparator, der mittels fernsteuerbarer Programmiermittel beeinflußbar ist, läßt sich die jeweils erreichbare Spannung des Ladekondensators den Anforderungen an die Stimulationsamplitude im jeweiligen Betriebszustand des Schrittmachers anpassen. Damit ist der Energieverbrauch zusätzlich dadurch optimiert, daß jeweils nur diejenige Spannung zur Verfügung gestellt wird, welche den tatsächlichen Erfordernissen entspricht. Diese Vorteile lassen sich insbesondere bei sogenannten "programmierbaren" Herzschrittmachern ausnutzen, welche die Vorgabe einer Vielzahl von Stimulationsamplituden zulassen.

Besonders vorteilhaft ist die erfindungsgemäße Schaltung dann, wenn sie in CMOS-Technologie hergestellt wird, wobei die Energieersparnis aufgrund der Tatsache, daß jeweils nur die zur Erzeugung des gewünschten Stimulationsimpulses tatsächlich notwendige Amplitude der Versorgungsspannung bereitgestellt wird, zusätzliche Vorteile erbringt, zumal bei dieser Herstellungstechnik die Verlustströme quadratisch mit der Spannung ansteigen.

Alternativ kann bei der erfindungsgemäßen Lösung bei Erreichen einer vorgegebenen Soll-Ausgangsspannung sowohl die Abgabe von Taktimpulsen an die Vervielfacherschaltung als auch diese Schaltung selbst intern inhibiert werden. Im erstgenannten Fall lassen sich - ausgehend von einer einzigen Vervielfacherschaltung - Ladungsimpulse für mehrere jeweils für sich auf unterschiedlichem Spannungsniveau regelbare Stimulationsausgangsstufen entnehmen. Bei programmierbaren Schrittmachern können dabei beispielsweise Atrium und Ventrikel mit unterschiedlicher Amplitude stimuliert werden.

Bei bevorzugten Weiterbildungen der Erfindung, wie sie auch in den Unteransprüchen angegeben sind, läßt sich bei geeigneter Wahl der Spanungsvervielfachung bzw. der wahlweisen Einschaltung der zusätzlichen Vervielfacher- bzw. Verdopplerschaltung in die Impulsausgangsstufe noch ein weiterer Vorteil dadurch erzielen, daß die Aufteilung in Schaltungsbereiche, die in "Low-voltage"-Technologie erstellt werden und solche die in "High-voltage"-Technologie erzeugt werden, in einer die Gesamtenergiebilanz der Schaltung begünstigen den Weise möglich ist.

Bei einer anderen Weiterbildung der Erfindung wird die Vervielfacherschaltung aus einer Schaltung mit zwei Schaltzuständen zur wahlweisen Erzeugung von Impulsen mit gegenüber der Versorgungsspannung erhöhter bzw. verminderter Spannung gebildet, wobei ein weiteres Schaltelement vorgesehen ist, welches bei seiner Aktivierung die Schaltung zur wahlweisen Erzeugung von Impulsen in Abhängigkeit von der Amplitude der zu erzeugenden Ausgangsimpulse in den entsprechenden der beiden Zustände setzt. Bevorzugt enthält dabei die Schaltung mit zwei Schaltzuständen eine Induktivität, die im Falle der Erzeugung von Impulsen mit verminderter Spannung taktgesteuert Versorgungsspannung und Ladekondensator leitend verbindet, während im Falle der Erzeugung von Impulsen mit erhöhter Spannung die Ladeimpulse mittels der in der Induktivität gespeicherten Energie nach Abschaltung des diese hervorrufenden Stroms generiert werden.

Ein bevorzugtes Ausführungsbeispiel der Erfindung ist in den Figuren dargestellt und wird nachfolgend anhand der Figuren näher beschrieben. Es zeigen:

Figur 1 ein Blockschaltung der Ausgangsstufe des erfindungsgemäßen Herzschrittmachers,

Figuren 2 bis 4 Einzelheiten gemäß Figur 1,

Figur 5 Impulsdiagramme, welche den zeitlichen Verlauf verschiedener Signale in der Schaltung gemäß Figur 1 wiedergeben, sowie

Figur 6 eine Einzelheit einer weiteren vorteilhaften Ausführungsform der Erfindung mit einem eine Induktivität enthaltenden Schaltregler.

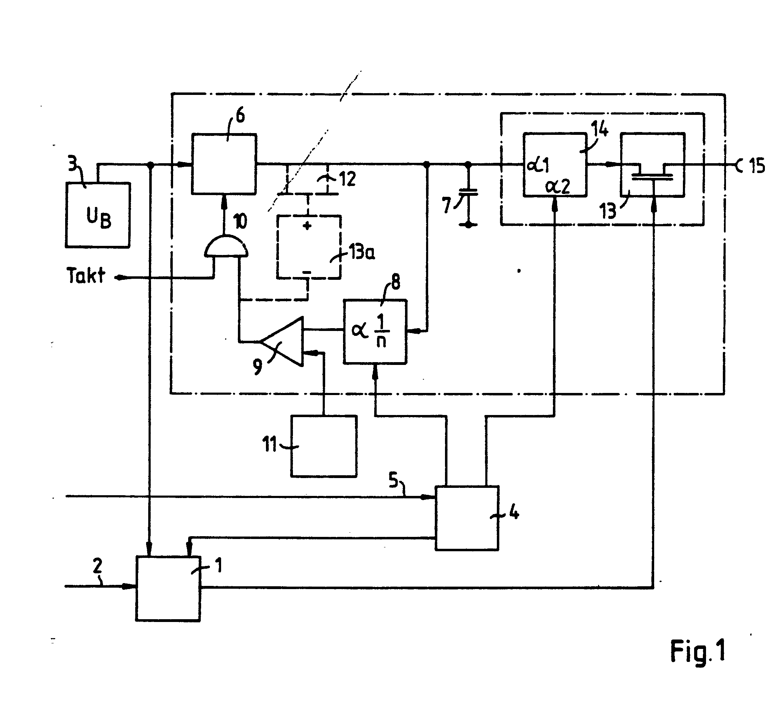

Bei dem in Figur 1 dargestellten Ausführungsbeispiel der Erfindung bildet ein Steuerlogikteil 1 diejenige Baugruppe, in der darüber entschieden wird, zu welchen Zeitpunkten Stimulationsimpulse abgegeben werden sollen. Das Steuerlogikteil wird dabei beeinflußt durch vom Herzen aufgenommene Signale, welche durch einen Pfeil 2 symbolisiert sind. Das Steuerlogikteil 1 enthält seine Versorgungsspannung aus einem Stromversorgungsteil 3, welches bei einem implantierbaren Schrittmacher bevorzugt durch Lithium-Batterien gebildet wird.

Als weitere bekannte in Schrittmachern verwendete Baugruppe ist ein Programmierteil 4 vorgesehen, welches durch von außerhalb übertragene Signale (Pfeil 5) die anderen Baugruppen des Schrittmachers in verschiedene Betriebszustände setzt. Unter anderem läßt sich durch den Programmierteil 4 die Reaktion des Steuerlogikteils 1 auf verschiedene Eingangssignale 2 beeinflussen.

Innerhalb der strichpunktierten Linie in Figur 1 sind die Bauelemente der erfindungsgemäßen "spannungsgeregelten" Ausgangsstufe wiedergegeben.

Mit der in Figur 1 dargestellten Ausgangsstufe sind Stimulationsimpulse programmierbar zu erzeugen, wobei die Stimulationsamplitude in einem weiten Bereich unabhängig von der Versorgungsspannung ist.

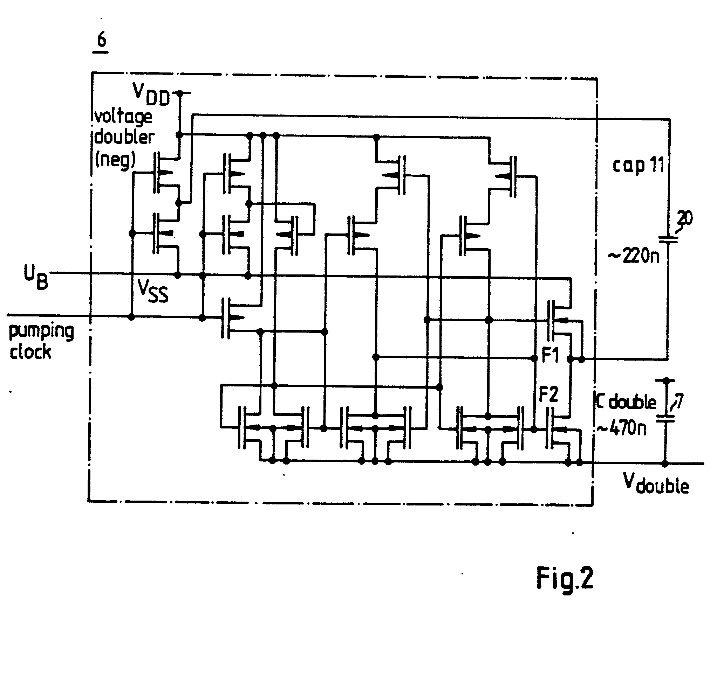

Die Batteriespannung wird einer integrierten CMOS-Spannungsverdopplerstufe 6 zugeführt, wie sie in Figur 2 noch einmal separat wiedergegeben ist. Dem in Figur 2 dargestellten Spannungsverdoppler werden an seinem Eingang ("pumpingclock") Taktimpulse zugeführt, welche dazu führen, daß an seinem Ausgang ("V-double") an dem dort befindlichen Ladekondensator 7 eine Spannung ansteht, die gegenüber dem Wert der Spannung $U_B$ ungefähr verdoppelt ist.

Ein Spannungsverdoppler läßt sich - im Gegensatz zu einem beliebigen Spannungsvervielfacher - relativ einfach in CMOS-Technik realisieren. Die in Figur 2 dargestellte Schaltung besteht (von links nach rechts) aus sogenannten "Level-Shiftern", zwei NOR-Gattern, zwei n-Kanal-MOS-FET-Schaltern und zwei externen Kondensatoren. Die Level-Shifter, welche aus einem Inverter und einem Flip-Flop bestehen, verwandeln die Spannung $U_B$ in die verdoppelte Spannung "U-double". Die NOR-Gatter erzeugen ein nichtüberlappendes Taktsignal, so daß die beiden FET-Schalter jeweils nicht gleichzeitig aktiviert werden können. Mittels der FET-Schalter wird die Ladung des Kondensators 20 in den Kondensator 7 überführt, welcher die Ausgangsspannung glättet. Der Wirkungsgrad dieser Schaltung ist ausgezeichnet, so daß der Energieverbrauch der Ausgangsschaltung insgesamt gering gehalten werden kann.

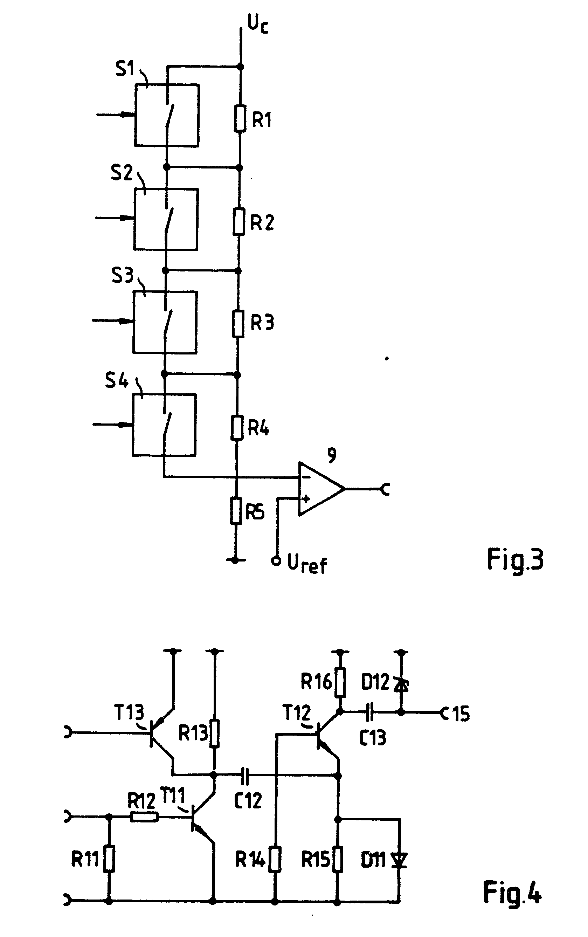

Die Ladespannung des Kondensators 7 wird einem programmierbaren Spannungsteiler 8 zugeführt, der einschließlich einem Komparator 9 in Figur 3 separat dargestellt ist. Die Spannung am Kondensator 7 wird durch eine Widerstandsteilerkette mit Widerständen R1 bis R5 an der Verbindungsstelle zwischen den Widerständen R4 und R5 dem invertierenden Eingang des Komparators 9 zugeführt, wobei die Widerstände R1 bis R4 durch Schalter S1 bis S4 in Abhängigkeit von den Programmsteuermitteln 4 (Figur 1) einzeln oder gemeinsam überbrückbar sind, so daß dem Komparator 9 ein variabler Anteil der Spannung $U_C$ zugeführt wird. Entsprechend ist diese Schaltung auch durch eine komplementäre Parallelschaltung von Widerständen realisierbar.

Die Werte der Widerstände R1 bis R4 bilden bevorzugt eine Reihe, die sich jeweils um den Faktor 2 unterscheidet, so daß durch entsprechende Betätigung der Schalter S1 bis S4 das Spannungsteilerverhältnis entsprechend binären Zahlenwerten durch entsprechende logische Schaltzustände der Programmsteuermittel direkt einstellbar sind.

Je nach Einstellung der Schalter S1 bis S4 wechselt das Ausgangssignal des Komparators 9, dessen positiver Eingang mit einer festen Referenzspannungsquelle verbunden ist, dann seine Polarität, wenn die Eingangsspannung $U_C$ einen Spannungspegel passiert, der dem durch die Eingangswiderstände eingestellten Verhältnis entspricht.

Das Ausgangssignal des Komparators 9 wird einem UND-Gatter 10 zugeführt, welches das Taktsignal für die Verdoppler-Schaltung 6 nur dann passieren läßt, wenn die Eingangsspannung am invertierenden Eingang des Komparators kleiner ist als die Spannung der Referenzspannungsquelle 11.

Bei einer alternativen Ausführungsform gestrichelt dargestellt gelangt das Ausgangssignal statt zu dem UND-Gatter 10 (welches in diesem Fall entfällt) zu einem Schaltelement 12, das in der Ausgangsleitung des Spannungsverdoppler 6 angeordnet ist und dann leitend wird, wenn die Spannung am Kondensator 7 als Versorgungsspannung für die Stimulationsausgangsstufe einen vorgegebenen Wert unterschreitet. Zwischen Komparator 9 und Schaltelement 12 ist bei der Verwendung von CMOS-Schaltungen dann ein Level-Shifter 13a einzuschalten, wenn die Komparatorschaltung 9 (wie der Steuerlogikteil 1) in an die Spannung der Stromversorgung angepaßten "Low-voltage"-Technologie hergestellt ist, während die Spannungsverdopplerstufe und die ihr folgenden Stufen in "High-voltage"-Technologie erzeugt sind.

Dem Kondensator 7 nachgeschaltet ist eine Impulsausgangsstufe 13, welche in Einzelheiten in Figur 4 wiedergegeben ist. Zur Abgabe eines Stimulationsimpulses wird - der im übrigen über dem Widerstand R11 gesperrt gehaltene - Transistor T11 über einen Widerstand R12 angesteuert, woraufhin der in Basisschaltung betriebene Transistor T12 über den am Kollektorwiderstand R13 des Transistors T11 angeschlossenen Kondensator C12 über seinen Emitter angesteuert wird. Der Kollektorstrom bewirkt einen Spannungsabfall am Widerstand R16, der sich über den Kondensator C13 an die Ausgangsklemme 15 fortpflanzt. Die Zener-Diode D12 schützt die Ausgangsstufe vor bei Defibrillation oder Depolarisation auftretenden Spannungen.

Der Kondensator C12 wird mittels des Transistors T13 nach Abgabe des Stimulationsimpulses erneut aufgeladen, wenn die Stimulationsausgangsstufe im Impulsverdopplerbetrieb arbeitet. Wird der Kondensator C12 dagegen bei nichtleitendem Transistor T13 nur über den Widerstand R13 geringfügig aufgeladen, so erfolgt die Stimulation lediglich mit einfacher Amplitude.

Die Betriebsweise der Stimulationsausgangsstufe wird durch die Programmsteuermittel 4 beeinflußt, wobei der Transistor T13 die Funktion des in Figur 1 dargestellten Spannungsverdoppler-Steuerbausteins 14 übernimmt. Je nach Programm kann die Impulsabgabe entweder einfach oder verdoppelt erfolgen.

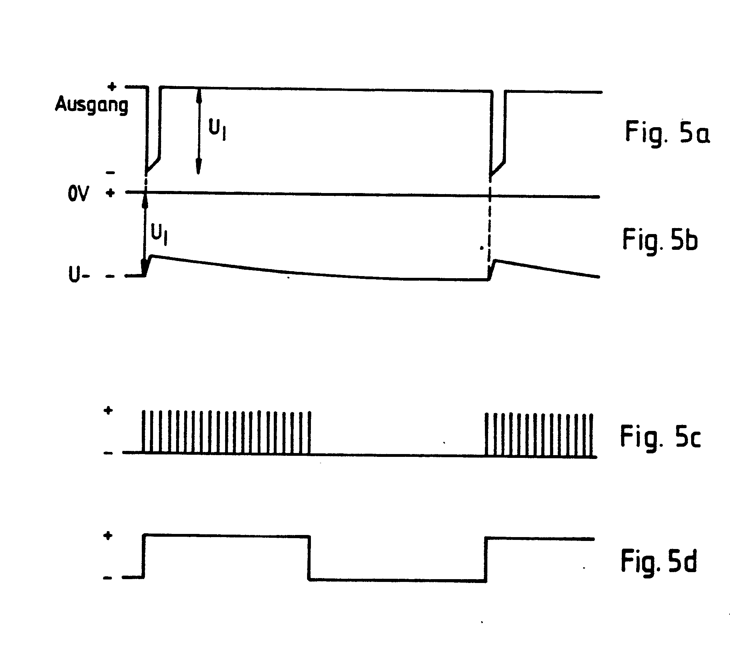

Die Impulsdiagramme in Figur 5 geben den Signalverlauf an verschiedenen Schaltungspunkten in Figur 1 wieder. Die Stimulationsimpulse am Ausgang sind dabei in Figur 5a wiedergegeben, während Figur 5b die Ladespannung am Kondensator 7 zeigt (jeweils bezogen auf den Pegel 0 Volt).

In Figur 5c sind die Ladeimpulse, welche den Kondensator 7 aufladen und von der Spannungsverdopplerstufe 6 abgegeben werden, dargestellt. Das Auftreten der Ladeimpulse wird vom Ausgangssignal des Komparators 9 beeinflußt, dessen Verlauf in Figur 5d wiedergegeben ist. Es ist ersichtlich, daß nach jedem Stimulationsimpuls jeweils eine Anzahl von Ladeimpulsen abgegeben wird, bis die Spannung am Kondensator 7 wieder den zur Abgabe des nachfolgenden Stimulationsimpulses notwendigen Wert erreicht hat. Die Versorgungsspannung für die Stimulationsausgangsstufe ist dabei jeweils vor Impulsabgabe konstant, wobei mit nachlassender Batteriespannung lediglich die Zahl der Ladeimpulse größer wird, welche erforderlich ist, um den Sollwert der Spannung des Kondensators 7 zu erreichen.

Dadurch, daß die Spannungsverdopplung der Stimulations-Ausgangsstufe wahlweise aktivierbar ist, läßt sich die Variation des Spannungsbereiches für die Ladung des Kondensators 7 in einem eingeschränkten Bereich halten, was für die verwendeten CMOS-Schaltungen günstig ist, da diese als Digitalschaltungen einerseits nur in einem begrenztem Spannungsbereich von Logikpegeln arbeiten und andererseits eine zu starke Vergrößerung der Versorgungs- und Eingangsspannungen zu einer relativ großen Wirkungsgradverschlechterung führt, da die Verlustströme mit dem Quadrat der Versorgungsspannung ansteigen. Mit der dargestellten Konfiguration ist es damit möglich, trotz eines eingeschränkten Variationsbereiches der mittels der Komparatorschaltung 9 beeinflußten Spannungsbereich des Ladekondensators 7 einen doppelt so großen Spannungsbereich der Stimulationsimpulse am Ausgang 15 zu kontrollieren.

Bei einem Schrittmacher mit getrennten Ausgangsschaltungen zur Stimulation in Atrium und Ventrikel ist die in Figur 1 strichpunktiert umrahmte Schaltung zweifach vorhanden, wobei die außerhalb dieser Linie befindlichen Elemente 1 bis 4 sowie die Referenzspannungsquelle 11 nur einfach vorgesehen sein müssen, so daß die Versorgung mit Ladungsimpulsen mit gegenüber der Versorgungsspannung heraufgesetzter Spannung doppelt ausgenutzt wird, während die Regelungs- und Impulsausgangsstufen getrennt vorhanden sind.

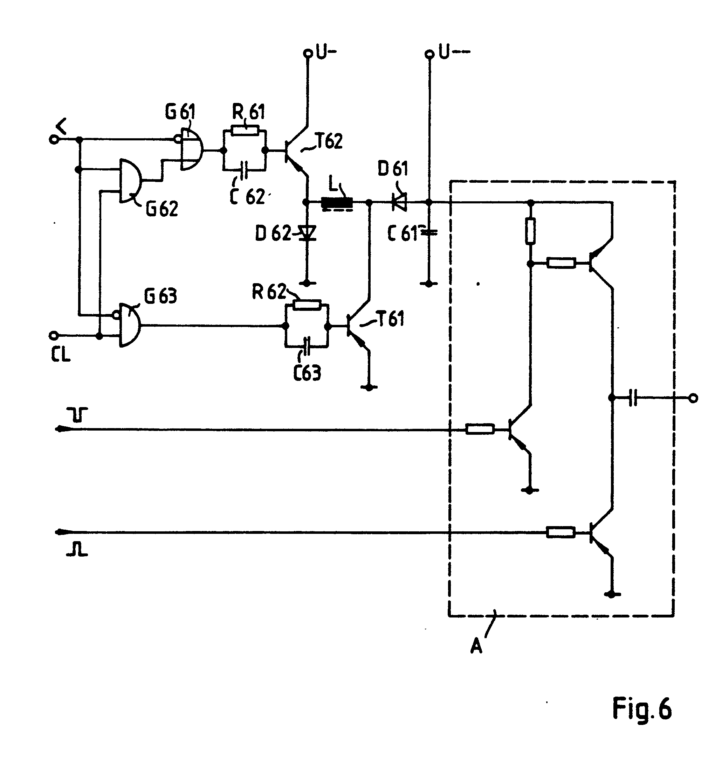

In der weiteren Figur 6 ist eine Schaltung dargestellt, welche eine vorteilhafte Regelung in einem Bereich einer Ausgangsspannung zwischen 0 Volt und einem über der Batteriespannung liegenden Spannungswert mit günstiger Leistungsbilanz ermöglicht. Dabei wird eine Induktivität L verwendet, deren gespeicherte magnetische Energie auf einen Ladekondensator C61 überführt wird. Je nachdem, ob der gewünschte Wert der Ausgangsspannung über oder unter der Batteriespannung U- liegt, werden einem Transistor T61 (Aufwärtsregelung) oder einem Transistor T62 (Abwärtsregelung) Taktimpulse zugeführt. Parallelschaltungen von je einem Widerstand R61 bzw. R62 und einem Kondensator C62 bzw. C63 im Eingang der Transistoren bewirken eine Verbesserung der Steilheit der Ansteuerimpulse.

Bei der Abwärtsregelung wird der Kondensator C61 direkt über den Transistor T62 pulsweise aufgeladen. Eine umgekehrt zur Flußrichtung geschaltete Diode D62 löscht dabei die bei der Spannungsabschaltung jeweils entstehenden Spannungsspitzen.

Im Falle der Schaltung als Aufwärtsregler wird als Vervielfacherschaltung ein Schaltregler mit Induktivität benutzt, wobei hier die bei Abschaltung des Stromflusses durch die Induktivität L hindurch dort entstehende Spannungsspitze zur Aufladung des Kondensators dient. Dabei wird bei dauernd eingeschaltetem Tansistor T62 der Transistor T61 zunächst kurzzeitig leitend geschaltet, so daß durch die Induktivität ein Strom zu fließen beginnt, der eine zeitlich zunehmende Tendenz hat. Bei Abschaltung des Transistors T61 baut sich an der Induktivität L aufgrund der gespeicherten magnetischen Energie eine entgegengesetzt gerichtete Spannung auf, welche bewirkt, daß eine elektrische Ladung in den Kondensator C61 fließt. Eine Diode D61 verhindert, daß bei gegenüber der am Kondensator C61 anstehenden Spannung verminderter Eingangsspannung ein Strom vom Kondensator C61 in die Ladeschaltung zurückfließt. Am Kondensator C61 steht damit die geregelte Spannung U-- zur Verfügung.

Die Taktimpulse werden stets über den Eingang CL zugeführt. Ist die Sollspannung U-- kleiner als die Versorgungsspannung U-, so ist der Eingang "<" aktiviert. Damit gelangen die Taktimpulse über ein UND-Gatter G62 und ein diesem nachgeschaltetes ODER-Gatter G61 an die Basis des Transistors T62, der wie zuvor beschrieben arbeitet. Ist dagegen die Sollausgangsspannung größer, so bleibt der Eingang "<" inaktiv. Der invertierende Eingang des ODER-Gatters G61 aktiviert aber des Ausgang und schaltet den Transistor T62 permanent durch. Das UND-Gatter G63 wird über seinen invertierenden Eingang für Taktimpulse durchlässig, welche den Transistor T61 in der dargestellten Weise ansteuern.

Die Anschlußweise des Komparators und der nachfolgenden Ausgangsschaltung A zur Erzeugung von Stimulationsimpulsen des Schrittmachers entspricht im Grundsatz derjenigen, wie sie anhand des zuvor dargestellten Ausführungsbeispiels beschrieben ist. Die Taktimpulse am Eingang CL, werden gesperrt, sobald die Spannung U-- am Kondensator C61 die Sollspannung erreicht. Je nachdem, ob die Sollspannung größer oder kleiner ist als die Versorgungsspannung U-, wird die Betriebsweise der Schaltung über den Eingang "<" entsprechend geändert. Diese Änderung erfolgt in Abhängigkeit vom Programmierungszustand des Schrittmachers.

Die Impulsausgangsschaltung A ist als Gegentaktschaltung ausgeführt, wobei der Spannungszustand des Ausgangskondensators durch die beiden dargestellten Ansteuerungseingänge bestimmt wird.

**Patentansprüche**

1. Herzschrittmacher bzw. ein vergleichbares Elektrostimulationsgerät mit einer impulsweise getakteten Spannungsvervielfacherschaltung (6) zum Erzeugen von Stimulationsimpulsen mit veränderbarer, insbesondere gegenüber der Versorgungsspannung heraufgesetzter, Spannung bzw. ein vergleichbares Elektrostimulationsgerät, mit einem Schaltelement (10, 12), welches bei seiner Aktivierung von der Vervielfacherschaltung (6) abgegebene getaktete Ladeimpulse mit gegenüber der Versorgungsspannung veränderter Spannung zu einem Ladekondensator (7) für die Versorgung der Ausgangsschaltung zur Erzeugung von Stimulationsimpulsen gelangen läßt, wobei das Schaltelement (10, 12) mittels eines Spannungskomparators inaktiv geschaltet wird, wenn die Spannung des Ladekondensators (7) einen vorgegebenen Wert überschreitet, dadurch gekennzeichnet, daß die getaktete Spannungsvervielfacherschaltung (6) so ausgebildet ist, daß die Variation des Spannungsbereichs für die Ladung des Ladekondensators (7) innerhalb des durch die Spannungspegel der verwendeten Digitalschaltungen eingeschränkten Bereichs bleibt und daß in die Ausgangsschaltung zur Erzeugung der Stimulationsimpulse eine weitere stufenhaft veränderliche Vervielfacherschaltung (14) einbezogen ist.

2. Herzschrittmacher bzw. ein vergleichbares Elektrostimulationsgerät nach Anspruch 1, dadurch gekennzeichnet, daß die weitere Vervielfacherschaltung (14) eine Endstufe mit einem Endstufentransistor mit Emitter- (bzw. Source-)kopplung enthält.

3. Herzschrittmacher bzw. ein vergleichbares Elektrostimulationsgerät nach einem der vorangehenden Ansprüche, dadurch gekennzeichnet, daß das Schaltelement (10, 12) durch eine Komparatorschaltung (9) angesteuert wird, der als Eingangssignal eine von der Spannung des Ladekondensators (7) vor der weiteren Spannungsvervielfacherschaltung (14) abgeleitete Spannung und eine feste Referenzspannung zugeführt werden.

4. Herzschrittmacher bzw. ein vergleichbares Elektrostimulationsgerät nach einem der vorangehenden Ansprüche, dadurch gekennzeichnet, daß mindestens eine der Vervielfacherschaltungen (6, 14) programmierbar mit unterschiedlichen Vervielfachungsfaktoren ausgebildet ist.

5. Herzschrittmacher bzw. ein vergleichbares Elektrostimulationsgerät nach Anspruch 3, dadurch gekennzeichnet, daß die Ableitung der Eingangsspannung des Komparators (9) von der Spannung des Ladekondensators (7) mittels eines programmierbaren Spannungsteilers (8) erfolgt.

6. Herzschrittmacher bzw. ein vergleichbares Elektrostimulationsgerät nach einem der vorangehenden Ansprüche, dadurch gekennzeichnet, daß die getaktete Vervielfacherschaltung und mindestens Teile der weiteren Vervielfacherschaltung in integrierter Form mittels CMOS-Technologie erzeugt ist, wobei die Endstufe in sogenannter "High-Voltage-" und die Vorstufen in sogenannter "Low-Voltage-" Technologie erzeugt sind.

7. Herzschrittmacher bzw. ein vergleichbares Elektrostimulationsgerät nach Anspruch 6, dadurch gekennzeichnet, daß der getakteten Vervielfacherstufe mindestens ein "Level-Shifter" (13a) nachgeschaltet ist.

8. Herzschrittmacher bzw. ein vergleichbares Elektrostimulationsgerät nach einem der vorangehenden Ansprüche, dadurch gekennzeichnet, daß der getakteten Vervielfacherschaltung (6) getrennt zwei Gruppen von Bauelementen jeweils bestehend aus einem Ladekondensator mit Ausgangsstufe, Schaltelement und Komparator nachgeschaltet sind, wobei die eine Gruppe von Bauelementen zur Regelung der Versorgungsspannung für die Stimulation des Atriums und die andere zur Regelung der Versorgungsspannung für die Stimulation des Ventrikels dient.

9. Herzschrittmacher bzw. ein vergleichbares Elektrostimulationsgerät nach Anspruch 8, dadurch gekennzeichnet, daß für die Stimulation des Atriums und des Ventrikels der jeweilige Komparator getrennt programmierbar und/oder eine getrennte Effektivitätskontrolle durchführbar ist.

10. Herzschrittmacher bzw. ein vergleichbares Elektrostimulationsgerät nach einem der vorangehenden Ansprüche, dadurch gekennzeichnet, daß die Komparatorschaltung (9) in Abhängigkeit von mittels Programmierungsmitteln vorgegebenen Werten und/oder vom Ergebnis einer Effektivitätskontrolle bezüglich der abgegebenen Stimulationsimpulse beeinflußt wird.

11. Herzschrittmacher bzw. ein vergleichbares Elektrostimulationsgerät nach einem der vorangehenden Ansprüche, dadurch gekennzeichnet, daß die getaktete Vervielfacherschaltung (6) aus einer Schaltung mit zwei Schaltzuständen zur wahlweisen Erzeugung von Impulsen mit gegenüber der Versorgungsspannung erhöhter bzw. verminderter Spannung besteht, wobei ein weiteres Schaltelement vorgesehen ist, welches bei seiner Aktivierung die Schaltung zur wahlweisen Erzeugung von Impulsen in Abhängigkeit von der Amplitude der zu erzeugenden Ausgangsimpulse in den entsprechenden der beiden Zustände setzt.

12. Herzschrittmacher bzw. ein vergleichbares Elektrostimulationsgerät nach Anspruch 11, dadurch gekennzeichnet, daß die Schaltung mit zwei Schaltzuständen eine Induktivität (L) enthält, die im Falle der Erzeugung von Impulsen mit verminderter Spannung taktgesteuert Versorgungsspannung (U-) und Ladekondensator (C61) leitend verbindet, während im Falle der Erzeugung von Impulsen mit erhöhter Spannung die Ladeimpulse mittels der in der Induktivität (L) gespeicherten Energie nach Abschaltung des diese hervorrufenden Stroms generiert werden.

13. Herzschrittmacher bzw. ein vergleichbares Elektrostimulationsgerät nach Anspruch 12, dadurch gekennzeichnet, daß die Induktivität (L) mittels eines erste elektronischen Schalters (T62) mit der Spannungsquelle (U-) verbindbar ist, während ein weiterer, mit dem anderen Anschluß verbundener elektronischer Schalter (T61) mindestens mittelbar parallel zu dem Ladekondensator (C61) in einen leitenden Zustand schaltbar ist, wobei der erste elektronische Schalter bei geöffnetem zweiten elektronischen Schalter impulsweise aktivierbar ist, während der zweite elektronische Schalter bei geschlossenem ersten elektronischen Schalter impulsweise aktivierbar ist.

**Claims**

1. A cardiac pacemaker or a comparable electrostimulation device having a voltage multiplier circuit (6), clocked pulsewise, to produce stimulation pulses with a variable voltage, particularly a voltage which is increased in comparison with the supply voltage, having a circuit element (10,12) which, on being activated, allows clocked charging pulses delivered by the multiplier circuit (6) to reach a charging capacitor (7) for supplying the output circuit for the production of stimulation pulses, with a voltage which is altered in comparison with the supply voltage, the circuit element (10, 12) being rendered inactive by means of a voltage comparator if the voltage of the charging capacitor (7) exceeds a preset value, characterised in that the clocked voltage multiplier circuit (6) is so constructed that the variation in the voltage range for the charging of the charging capacitor (7) remains within the range restricted by the voltage level of the digital circuits used and that a further multiplier circuit (14), which is variable in steps, is included in the output circuit for the production of the stimulation pulses.

2. A cardiac pacemaker or a comparable electrostimulation device as claimed in Claim 1, characterised in that the further multiplier circuit (14) contains an output-stage with an output-stage transistor with emitter (or source) coupling.

3. A cardiac pacemaker or a comparable electrostimulation device as claimed in any one of the preceding Claims, <u>characterised in that</u> the circuit element (10, 12) is actuated by a comparator circuit (9) to which a voltage derived from the voltage of the charging capacitor (7) before the further voltage multiplier circuit (14) and a fixed reference voltage are supplied as input signal.

4. A cardiac pacemaker or a comparable electrostimulation device as claimed in any one of the preceding Claims, <u>characterised in that</u> at least one of the multiplier circuits (6, 14) is made programmable with different multiplication factors.

5. A cardiac pacemaker or a comparable electrostimulation device as claimed in Claim 3, <u>characterised in that</u> the derivation of the input voltage of the comparator (9) from the voltage of the charging capacitor (7) is effected by means of a programmable voltage divider (8).

6. A cardiac pacemaker or a comparable electrostimulation device as claimed in any one of the preceding Claims, <u>characterised in that</u> the clocked multiplier circuit and at least parts of the further multiplier circuit are produced in integrated form by means of CMOS technology, the output stage being produced in so-called "high-voltage" technology and the preliminary stages being produced in so-called "low-voltage" technology.

7. A cardiac pacemaker or a comparable electrostimulation device as claimed in Claim 6, <u>characterised in that</u> the clocked multiplier stage is followed by at least one "level shifter" (13a).

8. A cardiac pacemaker or a comparable electrostimulation device as claimed in any one of the preceding Claims, <u>characterised in that</u> the clocked multiplier circuit (6) is followed separately by two groups of components each consisting of a charging capacitor with output stage, circuit element and comparator, the one group of components serving to control the supply voltage for the stimulation of the atrium and the other to control the supply voltage for the stimulation of the ventricle.

9. A cardiac pacemaker or a comparable electrostimulation device as claimed in Claim 8, <u>characterised in that</u>, for the stimulation of the atrium and of the ventricle, the respective comparator can be programmed separately and/or a separate effectiveness check can be carried out.

10. A cardiac pacemaker or a comparable electrostimulation device as claimed in any one of the preceding Claims, <u>characterised in that</u> the comparator circuit (9) is influenced depending on values preset by means of programming means and/or on the result of an effectiveness check with regard to the stimulation pulses delivered.

11. A cardiac pacemaker or a comparable electrostimulation device as claimed in any one of the preceding Claims, <u>characterised in that</u> the clocked multiplier circuit (6) consists of a circuit with two switching states for the selective production of pulses with a voltage which is increased or reduced in comparison with the supply voltage, a further circuit element being provided which, on being activated, sets the circuit for the selective production of pulses in the appropriate one of the two states depending on the amplitude of the output pulses to be produced.

12. A cardiac pacemaker or a comparable electrostimulation device as claimed in Claim 11, <u>characterised in that</u> the circuit with two switching states contains an inductance (L) which, in the case of the production of pulses with reduced voltage, conductively connects supply voltage (U—) and charging capacitor (C61), in a clocked manner, while in the case of the production of pulses with increased voltage, the charging pulses are generated by means of the energy stored in the inductor (L) after the current causing this has been switched off.

13. A cardiac pacemaker or a comparable electrostimulation device as claimed in Claim 12, <u>characterised in that</u> the inductor (L) can be connected to the voltage source (U—) by means of a first electronic switch (T62), while a further electronic switch (T61), connected to the other terminal at least indirectly parallel to the charging capacitor (C61) can be switched into a conducting state, the first electronic switch being able to be activated pulsewise when the second electronic switch is open while the second electronic switch can be activated pulsewise when the first electronic switch is closed.

**Revendications**

1. Stimulateur cardiaque ou appareil de stimulation électrique comparable, comprenant un circuit multiplicateur de tension (6) rythmé par impulsions pour générer des impulsions de stimulation ayant une tension variable, en particulier une tension augmentée par rapport à le tension d'alimentation, un élément de commutation (10, 12) qui, lorsqu'il est rendu actif, laisse parvenir des impulsions de charge, délivrées per le circuit multiplicateur (6) et ayant une tension modifiée par rapport à la tension d'alimentation, jusqu'à un condensateur de charge (7) pour l'alimentation d'un circuit de sortie en vue de la génération d'impulsions de stimulation, l'élément de commutation (10, 12) étant commuté à l'état inactif au moyen d'un comparateur de tensions lorsque la tension sur le condensateur de charge (7) dépasse une valeur préfixée,

caractérisé en ce que

le circuit multiplicateur de tension (6) rythmé est réalisé de manière que la variation de la plage de tension pour la charge du condensateur de charge (7) reste à l'intérieur de la plage délimitée par les niveaux de tension des circuits numériques utilisés et qu'un circuit multiplicateur supplémentaire (14), variable par échelons, est incorporé dans le circuit de sortie pour la génération des impulsions de stimulation.

2. Stimulateur cardiaque ou appereil de stimulation électrique comparable selon la revendication 1, caractérisé en ce que le circuit multiplicateur supplémentaire (14) contient un étage final avec un transistor d'étage final à couplage d'émetteur (ou de source).

3. Stimulateur cardiaque ou appareil de stimulation électrique comparable selon une des revendications précédentes, caractérisé en ce que l'élément de commutation (10, 12) est piloté par un circuit comparateur (9) auquel sont appliquées, en tant que signal d'entrée, une tension dérivée de la tension du condensateur de charge (7), en amont du circuit multiplicateur de tension supplémentaire (14), et une tension de référence fixe.

4. Stimulateur cardiaque ou appareil de stimulation électrique comparable selon une des revendications précédentes, caractérisé en ce que l'un au moins des circuits multiplicateurs (6, 14) est réalisé pour pouvoir être programmé avec des facteurs de multiplication différents.

5. Stimulateur cardiaque ou appareil de stimulation électrique comparable selon la revendication 3, caractérisé en ce que la dérivation de la tension d'entrée du comparateur (9), à partir de la tension du condensateur de charge (7), s'effectue au moyen d'un diviseur de tension (8) programmable.

6. Stimulateur cardiaque ou appareil de stimulation électrique comparable selon une des revendications précédentes, caractérisé en ce que le circuit multiplicateur rythmé et des parties au moins du circuit multiplicateur supplémentaire sont réalisés sous forme intégrée selon la technologie CMOS, l'étage final étant produit selon la technologie "High-Voltage" et les étages préliminaires étant produits selon la technologie "Low-Voltage".

7. Stimulateur cardiaque ou appareil de stimulation électrique comparable selon la revendication 6, caractérisé en ce que le circuit multiplicateur rythmé est suivi d'au moins un changeur de niveeu (13a).

8. Stimulateur cardiaque ou appareil de stimulation électrique comparable selon une des revendications précédentes, caractérisé en ce que le circuit multiplicateur rythmé (6) est suivi par deux groupes séparés d'éléments, comprenant chacun un condensateur de charge avec un étage de sortie, un élément de commutation et un comparateur, l'un des groupes d'éléments servant à la régulation de la tension d'alimentation pour le stimulation de l'oreillette et l'autre groupe d'éléments servant à la régulation de la tension d'alimentation pour la stimulation du ventricule.

9. Stimulateur cardiaque ou appereil de stimulation électrique comparable selon la revendication 8, caractérisé en ce que les comparateurs pour la stimulation de l'oreillette et pour celle du ventricule sont programmables séparément et/ou peuvent être soumis à des contrôles d'efficacité séparés.

10. Stimulateur cardiaque ou appareil dè stimulation électrique comparable selon une des revendications précédentes, caractérisé en ce que le circuit comparateur (9) est influencé par des valeurs préfixées à l'aide de moyens de programmation et/ou par le résultat d'un contrôle d'efficacité en ce qui concerne les impulsions de stimulation délivrées.

11. Stimulateur cardiaque ou appareil de stimulation électrique comparable selon une des revendications précédentes, caractérisé en ce que le circuit multiplicateur rythmé (6) comprend un circuit présentent deux états de commutation pour la génération sélective d'impulsions dont la tension est augmentée par rapport à la tension d'alimentation ou d'impulsions dont la tension est abaissée par rapport à la tension d'alimentation, un élément de commutation supplémentaire étant prévu pour, lorsqu'il est rendu actif, faire passer ce circuit à l'un ou l'autre des deux états suivant l'amplitude que doivent avoir les impulsions de sortie à générer.

12. Stimulateur cardiaque ou appareil de stimulation électrique comparable selon la revendication 11, caractérisé en ce que le circuit présentant deux états de commutation contient une inductance (L) qui, en cas de génération d'impulsions dont la tension est abaissée, établit, sous la commande d'un signal rythmé, une liaison conductrice entre la tension d'alimentation (U-) et le condensateur de charge (C61), tandis que, en cas de généretion d'impulsions dont la tension est augmentée, les impulsions de charge sont générées, au moyen de l'énergie accumulée dans l'inductance (L), après la coupure du courant qui est à l'origine de cette accumulation d'énergie.

13. Stimulateur cardiaque ou appareil de stimulation électrique comparable selon la revendication 12, caractérisé en ce que l'inductance (L) peut être connectée au moyen d'un premier interrupteur électronique (T62) à la source de tension (U-), tandis qu'un interrupteur électronique supplémentaire (T61), relié à l'autre borne d'alimentation, peut être commuté à un état conducteur, en parallèle avec le condenseteur de charge (C61), au moins de façon indirecte, le premier interrupteur étant susceptible d'être rendu actif par impulsions lorsque le second interrupteur est ouvert, tandis que le second interrupteur peut être rendu actif par impulsions lorsque le premier interrupteur est fermé.

Fig.1

Fig.2

0 094 341

Fig.3

Fig.4

5

Ausgang

$U_I$

0V

$U_I$

U-

Fig. 5a

Fig. 5b

Fig. 5c

Fig. 5d

Fig. 6